(19) Europäisches Patentamt
European Patent Office
Office européen des brevets

(11) Veröffentlichungsnummer: **0 088 274**
**B1**

# (12) EUROPÄISCHE PATENTSCHRIFT

(45) Veröffentlichungstag der Patentschrift: **03.09.86**

(51) Int. Cl.⁴: **C 07 D 211/90**, C 07 D 409/12, C 07 D 401/12, A 61 K 31/44

(21) Anmeldenummer: **83101624.1**

(22) Anmeldetag: **21.02.83**

## (54) Neue 1,4-Dihydropyridine, Verfahren zu ihrer Herstellung und ihrer Verwendung in Arzneimitteln.

(30) Priorität: **05.03.82 DE 3207982**

(43) Veröffentlichungstag der Anmeldung: **14.09.83 Patentblatt 83/37**

(45) Bekanntmachung des Hinweises auf die Patenterteilung: **03.09.86 Patentblatt 86/36**

(84) Benannte Vertragsstaaten: **AT BE CH DE FR GB IT LI NL**

(56) Entgegenhaltungen:
**EP-A-0 000 150**
**EP-A-0 002 231**
**EP-A-0 012 180**
**EP-A-0 039 863**
**EP-A-0 060 897**
**DE-A-2 228 363**
**DE-A-2 405 658**
**DE-A-2 635 916**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Goldmann, Siegfried, Dr., Kuckuckstrasse 41, D-5600 Wuppertal 2 (DE)**
Erfinder: **Böshagen, Horst, Dr., Wiesenstrasse 4, D-5657 Haan (DE)**
Erfinder: **Stoltefuss, Jürgen, Dipl.- Ing., Parkstrasse 20, D-5657 Haan (DE)**
Erfinder: **Schramm, Mathias, Dr., Paffrather Strasse 38, D-5000 Köln 80 (DE)**
Erfinder: **Thomas, Günter, Dr., Claudiusweg 9, D-5600 Wuppertal 1 (DE)**
Erfinder: **Kazda, Stanislav, Dr., Gellertweg 18, D-5600 Wuppertal 1 (DE)**

**EP 0 088 274 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft neue 1,4-Dihydropyridine mehrere Verfahren zu ihrer Herstellung sowie ihre Verwendung in Arzneimitteln, insbesondere in kreislaufbeeinflussenden Arzneimitteln.

Es ist bereits bekannt geworden, daß man 1,4-Dihydro-2,6-dimethyl-4-phenyl-pyridin-3,5-dicarbonsäurediethylester erhält, wenn man Benzylidenacetessigsäureethylester mit β-Aminocrotonsäureethylester oder Acetessigsäureethylester und Ammoniak umsetzt (E. Knoevenagel, Ber. dtsch. chem. Ges. 31, 743 (1898)).

Weiterhin ist bekannt, daß bestimmte 1,4-Dihydropyridine interessante pharmakologische Eigenschaften aufweisen (F. Bossert, W. Vater, Naturwissenschaften 58, 578 (1971), DE-A-2 117 571).

Es sind ebenfalls einige Dihydropyridine bekannt, die am Phenylring in 4-Position andere Substituenten tragen, vergl. EP-A 60 897; EP-A 39 863; EP-A 12 180; EP-A 2 231; DE-A 2 635 916. Die erfindungsgemäßen Dihydropyridine, die sich durch die neuartige Substituentengruppe X-B-Y vom Stand der Technik unterscheiden, weisen eine überraschende und signifikante Wirkungssteigerung in der Blutdrucksenkung auf.

Die vorliegende Erfindung betrifft neue 1,4-Dihydropyridine der allgemeinen Formel I

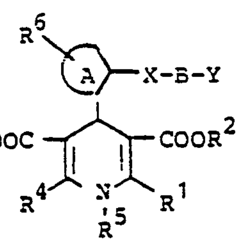

(I)

in welcher

$R^1$ und $R^4$ gleich oder verschieden sind und für Wasserstoff oder einen geradkettigen oder verzweigten Alkylrest mit bis zu 6 Kohlenstoffatomen stehen, der gegebenenfalls 1 oder 2 gleiche oder verschiedene Heterokettenglieder aus der Gruppe O, CO, NH oder N-Alkyl mit 1 bis 4 Kohlenstoffatomen enthält und der gegebenenfalls substituiert ist durch Halogen, Nitro, Cyano, Hydroxy, Amino oder Phenyl,

$R^2$ und $R^3$ gleich oder verschieden sind und jeweils für einen verzweigten oder unverzweigten Alkyloder Alkenylrest mit bis zu 6 Kohlenstoffatomen stehen, der gegebenenfalls substituiert ist durch Halogen, Hydroxy, Cyano, Amido, Monoalkylamino, Dialkylamino oder Alkyl-benzylamino, wobei die genannten Alkylreste jeweils 1 bis 4 Kohlenstoffatome enthalten,

$R^5$ für Wasserstoff, Benzyl oder einen geradkettigen oder verzweigten Alkylrest mit bis zu 6 Kohlenstoffatomen steht, der gegebenenfalls substituiert ist durch Fluor, Chlor oder Alkoxy mit 1 bis 4 Kohlenstoffatomen,

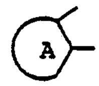

für Phenyl, Naphthyl, Thienyl, Furyl oder Pyridyl steht,

$R^6$ für einen oder 2 gleiche oder verschiedene Substituenten aus der Gruppe Wasserstoff, Alkyl, Alkoxy mit jeweils 1 bis 4 Kohlenstoffatomen, Fluor, Chlor, Brom, Cyano, Trifluormethyl, Carboxy, Amino, Hydroxy, Alkylamino oder Halogenalkoxy mit jeweils 1 bis 4 Kohlenstoffatomen steht,

X für ein Brückenglied aus der Gruppe O, S oder SO steht,

B für eine geradkettige oder verzweigte Alkylengruppe mit 1 bis 4 Kohlenstoffatomen steht und

Y für Phenyl, Pyridyl, Cycloalkyl mit 3 bis 7 Kohlenstoffatomen oder Alkenyl mit 6 bis 10 Kohlenstoffatomen steht, wobei diese Reste entweder direkt mit 8 verbunden sind oder über ein Heteroatom aus der Gruppe O, S, SO, $SO_2$, NH oder N-Alkyl mit 1 bis 4 Kohlenstoffatomen, mit B verbunden sind und wobei diese Reste gegebenenfalls 1 oder 2 gleiche oder verschiedene Substituenten aus der Gruppe Nitro, Halogen, Trifluormethyl, Cyano, Hydroxy, Phenyl, Alkyl und Alkoxy mit jeweils 1 bis 4 Kohlenstoffatomen enthalten,

sowie ihre pharmazeutisch unbedenklichen Additionssalze.

Die erfindungsgemäßen Verbindungen der allgemeinen Formel (I) können hergestellt werden indem man

A) Yliden-β-ketoester der allgemeinem Formel (II)

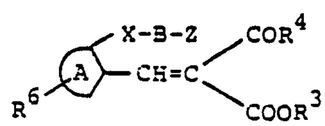

(I:

in welcher

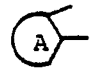 ,

$R^3$, $R^4$, $R^6$, X, B und Z die oben angegebene Bedeutung haben,

mit Enaminocarbonsäureestern der allgemeinen Formel (III)

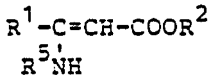

(III)

in welcher $R^1$, $R^2$ und $R^5$ die oben angegebene Bedeutung haben, gegebenenfalls in Gegenwart inerter organischer Lösungsmittel bei Temperaturen zwischen 20 und 150° C umsetzt, oder

B) Yliden-β-ketoester der allgemeinen Formel (II)

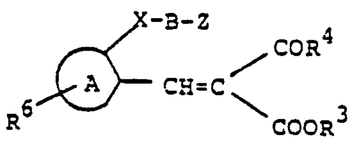

(II)

in welcher

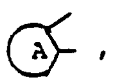

,

$R^3$, $R^4$, $R^6$, X, B und Z die oben angegebene Bedeutung haben, mit Aminen der allgemeinen Formel (IV)

$R^5-NH_2$ (IV)

in welcher $R^5$ die oben angegebene Bedeutung besitzt und β-Ketocarbonsäureestern der allgemeinen Formel (V)

$R^1-CO-CH_2-COOR^2$ (V)

in welcher $R^1$ und $R^2$ die oben angegebene Bedeutung haben, gegebenenfalls in Gegenwart inerter organischer Lösungsmittel bei Temperaturen zwischen 20 und 150° C umsetzt, oder

C) Aldehyde der allgemeinen Formel (VI)

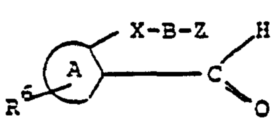

(VI)

in welcher

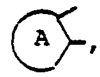

,

$R^6$, X, B und Z die oben angegebene Bedeutung haben, mit Enaminocarbonsäureestern der allgemeinen Formel (III)

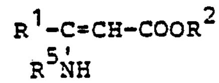

(III)

in welcher $R^1$, $R^2$ und $R^5$ die oben angegebene Bedeutung haben, und β-Ketocarbonsäureester der allgemeinen Formel (VII)

$R^4-CO-CH_2-COOR^3$ (VII)

in welcher $R^3$ und $R^4$ die oben angegebene Bedeutung haben, gegebenenfalls in Gegenwart inerter organischer Lösungsmittel bei Temperaturen zwischen 20 und 150° C umsetzt, oder

D) Aldehyde der allgemeinen Formel (VI)

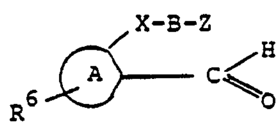

(VI)

in welcher

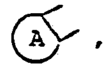

,

$R^6$, X, B und Z die oben angegebene Bedeutung haben, mit Aminen der allgemeinen Formel (IV)

$R^5-NH_2$ (IV)

in welcher $R^5$ die oben angegebene Bedeutung hat, und zwei äquivalenten β-Ketoester der allgemeinen Formel (VII)

$R^4-CO-CH_2-COOR^3$ (VII)

in welcher $R^3$ und $R^4$ die oben angegebene Bedeutung haben, gegebenenfalls in Gegenwart inerter organischer Lösungsmittel bei Temperaturen zwischen 20 und 150°C umsetzt.

Die neuen erfindungsgemäßen Dihydropyridine der allgemeinen Formel (I) besitzen wertvolle pharmazeutische Eigenschaften. Aufgrund ihrer kreislaufbeeinflussenden Wirkung können sie als antihypertensive Mittel, als Vasodilatatoren, als Cerebraltherapeutika sowie als

Coronartherapeutika Verwendung finden.

Bei Kenntnis des Standes der Technik war es nicht vorhersehbar, daß durch die Einführung des neuen Substituenten in 4-Position der Dihydropyridine Stoffe erhalten werden, die sich durch vorteilhafte biologische Eigenschaften auszeichnen. Aufgrund ihrer unerwarteten pharmazeutischen Wirkung stellen sie eine Bereicherung der Technik dar.

Je nach Art der eingesetzten Ausgangsstoffe kann die Synthese der erfindungsgemäßen Verbindungen der allgemeinen Formel (I) nach den einzelnen Verfahrensvarianten durch folgendes Formelschema wiedergegeben werden:

**Verfahrensvariante A**

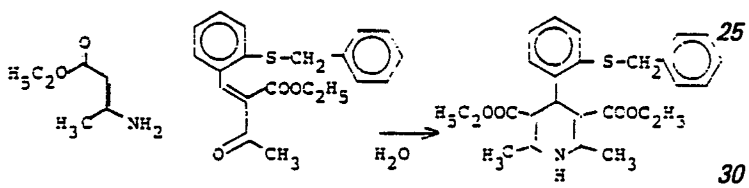


**Verfahrensvariante B**

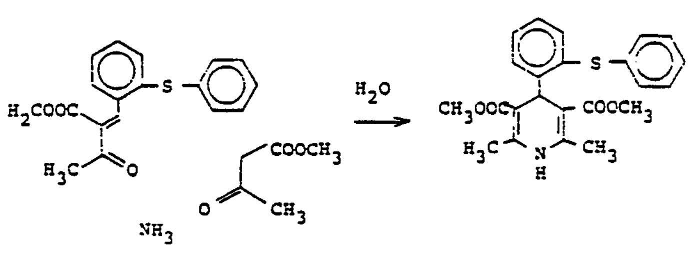


**Verfahrensvariante C**

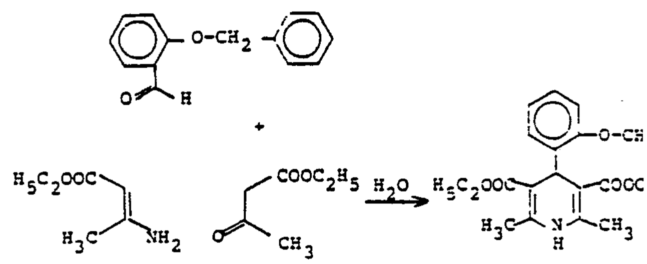


**Verfahrensvariante D**

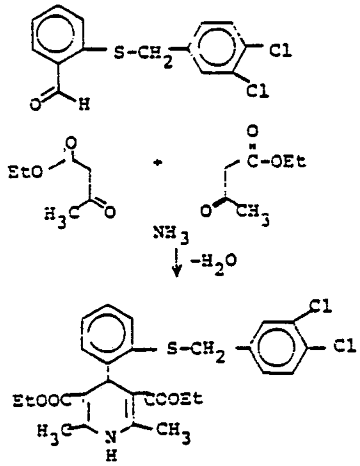


Die als Ausgangsstoffe verwendeten Yliden-β-ketoester der Formel (II) können nach literaturbekannten Methoden dargestellt werden (vgl. G. Jones, "The Knoevenagel Condensation", in Org. Reactions, Vol. XV, 204 ff (1967)).

Die als Ausgangsstoffe verwendeten Enaminocarbonsäureester (III) sind bekannt oder können nach literaturbekannten Methoden dargestellt werden (vgl. A.C. Cope, J. Am. Chem. Soc. 67, 1017 (1945)).

Die als Ausgangsstoffe verwendeten β-Ketocarbonsäureester (V) sind bekannt oder können nach literaturbekannten Methoden dargestellt werden (z.B. D. Borrmann, "Umsetzung von Diketen mit Alkoholen, Phenolen und Mercaptanen", in Houben-Weyl, Methoden der organischen Chemie, Vol. VII/4, 230 ff (1968) Y. Oikawa, K. Sugano und O. Yonemitsu, J. Org. Chem. 43, 2087 (1978)).

Die als Ausgangsstoffe verwendeten Aldehyde (VI) sind bekannt oder können nach literaturbekannten Methoden dargestellt werden (vgl. T.D. Harris und G.P. Roth, J. org. Chem. 44, 146 (1979)

DE-A- 2 165 260 (Juli 1972)

DE-A- 2 401 665 (Juli 1974)

Mijano et al, Chem. Abst. 59 (1963), 13 929 c, E. Adler und H.-D. Becker, Chem. Scand. 15, 849 (1961), E.P. Papadopoulos, M. Mardin und Ch. Issidoridis, J. Org. Chem. 31, 615 (1966) J. Am. chem. Soc. 78, 2543 (1956)).

Für alle Verfahrensvarianten A, B, C und D kommen als Verdünnungsmittel alle inerten organischen Lösungsmittel in Frage. Hierzu gehören vorzugsweise Alkohole wie Ethanol, Methanol, Isopropanol, Ether wie Dioxan, Diethylether, Tetrahydrofuran, Glykolmonomethylether, Glykoldimethylether oder Eisessig, Dimethylformamid, Dimethylsulfoxid, Acetonitril, Pyridin und Hexamethylphosphorsäuretriamid.

Die Reaktionstemperaturen können in einem größeren Bereich variiert werden. Im allgemeinen arbeitet man zwischen 20 und 150°C, vorzugsweise jedoch bei der Siedetemperatur des jeweiligen Lösungsmittels.

Die Umsetzung kann bei Normaldruck, aber auch bei erhöhtem Druck durchgeführt werden. Im allgemeinen arbeitet man unter Normaldruck.

Die vorstehenden Herstellungsverfahren sind lediglich zur Verdeutlichung angegeben, und die Herstellung der Verbindungen der Formel (I) ist nicht auf diese Verfahren beschränkt, sondern jede Modifikation dieser Verfahren ist in gleicher Weise für die Herstellung der erfindungsgemäßen Verbindungen anwendbar.

Je nach der Wahl der Ausgangssubstanzen können die erfindungsgemäßen Verbindungen in stereoisomeren Formen existieren, die sich entweder wie Bild und Spiegelbild (Enantiomere) oder die sich nicht wie Bild und Spiegelbild (Diastereomere) verhalten. Sowohl die Antipoden als auch die Racemformen als auch die Diastereomerengemische sind Gegenstand der vorliegenden Erfindung. Die Racemformen lassen sich ebenso wie die Deastereomeren in bekannter Weise in die stereoisomer einheitlichen Bestandteile trennen (vgl. z.B. E.L. Eliel, Stereochemistry of Carbon Compounds, McGraw Hill, 1962).

Die Verbindungen der allgemeinen Formel (I) zeigen interessante biologische Wirkungen. Sie besitzen ein breites und vielseitiges pharmakologisches Wirkungsspektrum. Im einzelnen seien folgende Hauptwirkungen genannt:

1. Die Verbindungen bewirken bei parenteraler, oraler und perlingualer Zugabe eine deutliche Erweiterung der Coronargefäße. Diese Wirkung auf die Coronargefäße wird durch einen gleichzeitigen nitritähnlichen herzentlastenden Effekt verstärkt.

Sie beeinflussen bzw. verändern den Herzstoffwechsel im Sinne einer Energieersparnis.

2. Die Erregbarkeit des Reizbildungs- und Erregungsleitungssystems innerhalb des Herzens wird herabgesetzt, so daß eine in therapeutischen Dosen nachweisbare Antiflimmerwirkung resultiert.

3. Der Tonus der glatten Muskulatur der Gefäße wird unter der Wirkung der Verbindungen stark vermindert. Diese gefäßspasmolytische Wirkung kann im gesamten Gefäßsystem stattfinden, oder sich mehr oder weniger isoliert in umschriebenen Gefäßgebieten (wiez.B. im Gehirn) manifestieren.

4. Die Verbindungen senken den Blutdruck von normotonen und hypertonen Tieren und können somit als antihypertensive Mittel verwendet werden.

5. Die Verbindungen haben stark muskulärspasmolytische Wirkungen, die an der glatten Muskulatur des Magens, Darmtraktes, des Urogenitaltraktes und des Respirationssystems deutlich werden.

Die neuen Wirkstoffe können in bekannter Weise in die üblichen Formulierungen übergeführt werden, wie Tabletten, Kapseln, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt:

Wasser, nicht-toxische organische Lösungsmittel, wie Paraffine (z.B. Erdölfraktionen), pflanzliche öle (z.B. Erdnuß-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermittel (z.B. Polyoxyethylen-Fettsäure-Ester, Polyoxyethylen-Fettalkohol-Ether, Alkylsulfonate

und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen und/oder Elixiere, die für orale Anwendungen gedacht sind, können die Wirkstoffe außer den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,01 bis 10 mg/kg, vorzugsweise etwa 0,01 bis 5 mg/kg Körpergewicht pro Tag zur Erzielung wirksamer Ergebnisse zu verabreichen, und bei oraler Applikation beträgt die Dosierung etwa 0,1 bis 20 mg/kg, vorzugsweise 0,5 bis 5 mg/kg Körpergewicht pro Tag.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. deren Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Im Falle der Applikation größerer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäß gelten hierbei auch die obigen Ausführungen.

**Beispiel 1**

4-(2-Benzylthiophenyl)-1,4-dihydro-2,6-dimethyl-Pyridin-3,5-dicarbonsäuredimethylester

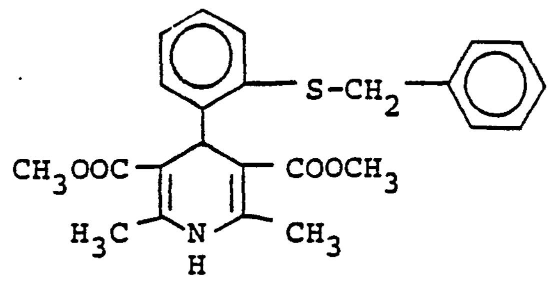

**Verfahrensvariante A**

Eine Lösung von 100 mmol (2-Benzylthio)benzylidenacetessigsäuremethylester und 100 mmol β-Amino-crotonsäuremethylester in 100 ml Methanol wird 24 h zum Sieden erhitzt.

Das Lösungsmittel wird im Vakuum abgezogen und der Rückstand mit wenig Ether kristallisiert.

FP: 84-90° C

Ausbeute: 25 %

**Beispiel 2**

4-(2-Benzyloxyphenyl)-1,4-dihydro-2,6-dimethylpyridin-3,5-dicarbonsäuredimethylester

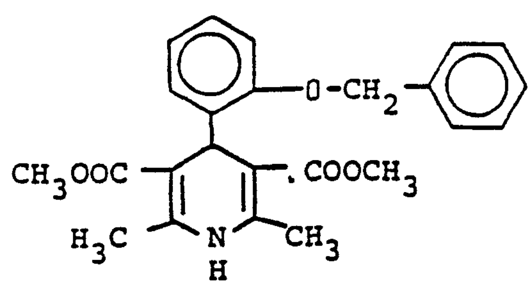

**Verfahrensvariante B**

Eine Lösung von 100 mmol (2-Benzyloxy)benzylidenacetessigsäuremethylester, 100 mmol Acetessigsäuremethylester und 120 mmol konzentrierte wäßrige Ammoniaklösung wurde 24 h in 100 ml Ethanol zum Sieden erhitzt.

Das Lösungsmittel wurde im Vakuum abgezogen und der Rückstand kristallisiert.

FP: 129-135° C

**Beispeiel 3**

2-Acetoxymethyl-4-(2-benzyloxyphenyl)-1,4-dihydro-6-methylpyridin-3,5-dicarbonsäurediethylester

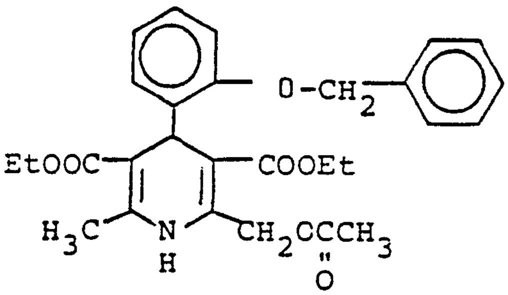

**Verfahrensvariante C**

100 mmol 2-Benzyloxybenzaldehyd, 100 mmol β-Aminocrotonsäureethylester und 100 mmol 4-Acetoxyacetessigsäureethylester wurden in 100 ml Ethanol 12 h am Rückfluß erhitzt.

Das Lösungsmittel wurde abgezogen und der Rückstand kristallisiert.

FP: 162-165°C.

**Beispiel 4**

1,4-Dihydro-2,6-dimethyl-4-/(2-phenylthiomethyloxy)-Phenyl/-3,5-dicarbonsäuredimethylester

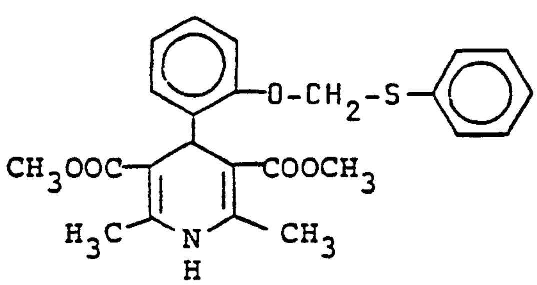

**Verfahrensvariante D**

100 mmol 2-(Phenylthiomethyloxy)phenylbenzaldehyd, 200 mmol Acetessigsäuremethylester und 120 mmol konzentrierte wäßrige Ammoniaklösung wurde in 100 ml Ethanol 12 h am Rückfluß gekocht, das Lösungsmittel abdestilliert und der Rückstand kristallisiert.

FP: 159°C

Analog den Beispielen 1-4 wurden dargestellt

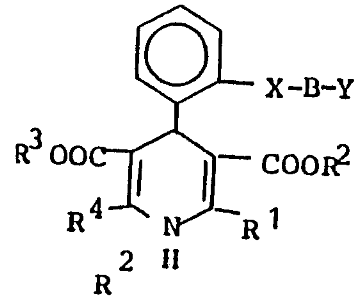

| Beispiel | $R^3$ | $R^4$ | $R^1$ | $R^2$ | X-B | Y | FP/°C | Ausbeute (Darstellungsverfahren) |
|---|---|---|---|---|---|---|---|---|
| 5 | $-C_2H_5$ | $-CH_2O\overset{O}{\overset{\|}{C}}-CH_3$ | $-CH_2O\overset{O}{\overset{\|}{C}}-CH_3$ | $-C_2H_5$ | $-O-CH_2-$ | 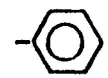 | 202-204 | 45 % (D) |
| 6 | $-C_2H_5$ | $-CH_2O\overset{O}{\overset{\|}{C}}-CH_3$ | $-CH_3$ | $-CH_3$ | $-O-CH_2-$ | 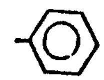 | 137 | 60 % (C) |
| 7 | $-CH_3$ | $-CH_3$ | $-CH_3$ | $-CH_3$ | -S- | 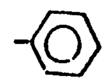 | 146 | 48 % (C) |
| 8 | $-CH_3$ | $-CH_3$ | $-CH_3$ | $-CH_3$ | $-S-CH_2-$ | 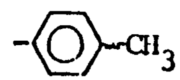 | 145-146 | 20 % (C) |
| 9 | $-C_2H_5$ | $-CH_3$ | $-CH_3$ | $-C_2H_5$ | $-S-CH_2-$ | 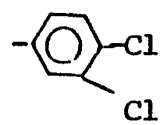 | 138 | 35 % (D) |

| Beispiel | $R^3$ | $R^4$ | $R^1$ | $R^2$ | X-B | Y | FP/°C | Ausbeute (Darstellungsverfahren) |
|---|---|---|---|---|---|---|---|---|
| 10 | $-C_2H_5$ | $-CH_3$ | $-CH_3$ | $-C_2H_5$ | $-S-CH_2$ | 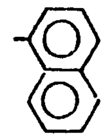 | amorph | 40 % (C) |
| 11 | $-CH_2-CH_2CN$ | $-CH_3$ | $-CH_3$ | $-CH_3$ | $-S-CH_2$ | 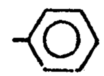 | 128-130 | 40 % (C) |
| 12 | $-C_2H_5$ | $-CH_3$ | $-CH_3$ | $-CH_3$ | $-S-CH_2$ | 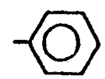 | 54-57 | 25 % (C) |
| 13 | $-(CH_2)_2-OCH_3$ | $-CH_3$ | $-CH_3$ | $-CH_3$ | $-S-CH_2$ | 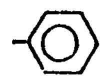 | 145-149 | 45 % (C) |
| 14 | " | $-CH_3$ | $-CH_3$ | $-C_2H_5$ | $-S-CH_2$ | 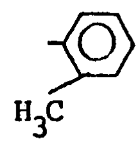 | 114-116 | 40 % (C) |
| 15 | $-(CH_2)_2-N(CH_3)_2$ | $-CH_3$ | $-CH_3$ | $-(CH_2)_2N(CH_3)_2$ | $-S-CH_2$ | 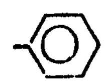 | 95-104 | 55 % (D) |
| 16 | $-C_2H_5$ | $-CH_2OC(=O)-CH_3$ | $-CH_3$ | $-C_2H_5$ | $-O-CH_2$ | 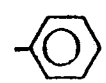 $-CH_3$ | 159-160 | 45 % (C) |

| Beispiel | $R^3$ | $R^4$ | $R^1$ | $R^2$ | X-B | Y | FP/°C | Ausbeute (Darstellungsverfahren) |
|---|---|---|---|---|---|---|---|---|
| 17 | $-(CH_2)_2-N(CH_3)_2$ | $-CH_3$ | $-CH_3$ | $-CH_3$ | $-S-CH_2$ | 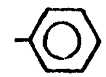 | 88-94 | 45 % (C) |
| 18 | $-(CH_2)_2CN$ | $-CH_3$ | $-CH_3$ | $-CH_3$ | $-S-CH_2$ | 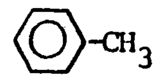 | 163-165 | 44 % (C) |
| 19 | $-C_2H_5$ | $-CH_2-NH_2$ | $-CH_3$ | $-C_2H_5$ | $-S-CH_2$ | 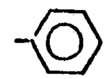 | 150 | 25 % (C) |
| 20 | $-CH_3$ | $-CH_3$ | $-CH_3$ | $-CH_3$ | $-O-CH_2$ | 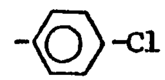 | 210 | 43 % (D) |
| 21 | $-CH_3$ | $-CH_3$ | $-CH_3$ | $-CH_3$ | $-O-CH_2$ | 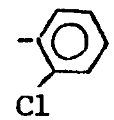 | 166 | 39 % (D) |
| 22 | $-CH_3$ | $-CH_3$ | $-CH_3$ | $-CH_3$ | $-O-CH_2$ | 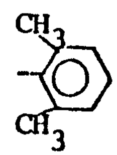 | 205 | 38 % (D) |
| 23 | $-CH_3$ | $-CH_3$ | $-CH_3$ | $-CH_3$ | $-O-CH_2$ | 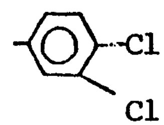 | 143 | 44 % (D) |
| 24 | $-CH_3$ | $-CH_3$ | $-CH_3$ | $-CH_3$ | $-O-CH_2$ | 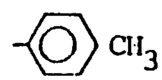 | 177 | 71 % (D) |

| Bei-spiel | $R^3$ | $R^4$ | $R^1$ | $R^2$ | X-B | Y | FP/°C | Ausbeute (Darstellungs-verfahren) |
|---|---|---|---|---|---|---|---|---|
| 25 | $-CH_3$ | $-CH_3$ | $-CH_3$ | $-CH_3$ | $-O-CH_2CH_2$ | 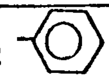 | 154 | 26 % (D) |
| 26 | $-CH_3$ | $-CH_3$ | $-CH_3$ | $-CH_3$ | $-O-CH_2$ | 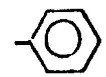 | 183 | 65 % (D) |
| 27 | $-CH_3$ | $-CH_3$ | $-CH_3$ | $-CH_3$ | $-O-CH_2-CH_2-O-$ | 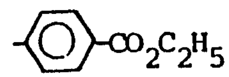 | 170 | 22 % (D) |
| 28 | $-CH_3$ | $-CH_3$ | $-CH_3$ | $-(CH_2)_2CN$ | $-O-CH_2-$ | 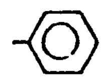 | ab 108 | 30 % (C) |
| 29 | $-CH_3$ | $-CH_3$ | $-CH_3$ | $-(CH_2)_2CN$ | $-O-CH_2-$ | 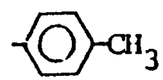 | 165 | 40 % (C) |
| 30 | $-CH_3$ | $-CH_3$ | $-CH_3$ | $-(CH_2)_2CN$ | $-O-CH_2-$ | 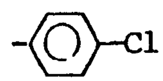 | 189 | 44 % (C) |
| 31 | $-CH_3$ | $-CH_3$ | $-CH_3$ | $-(CH_2)_2CN$ | $-O-CH_2-$ | 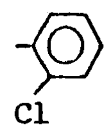 | 165 | 54 % (C) |

| Bei-spiel | $R^3$ | $R^4$ | $R^1$ | $R^2$ | X-B | Y | FP/°C | Ausbeute (Darstellungs-verfahren) |
|---|---|---|---|---|---|---|---|---|
| 32 | $-CH_3$ | $-CH_3$ | $-CH_3$ | $-(CH_2)_2CN$ | $-O-CH_2-$ | 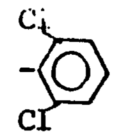 | 192–194 | 69 % (C) |
| 33 | $-CH_3$ | $-CH_3$ | $-CH_3$ | $-(CH_2)_2CN$ | $-OCH_2CH_2-$ | 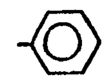 | 163–164 | 54 % (C) |
| 34 | $-CH_3$ | $-CH_3$ | $-CH_3$ | $-(CH_2)_2CN$ | $-O-CH_2-$ | 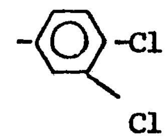 | 191–192 | 46 % (C) |
| 35 | $-CH(CH_3)_2$ | $-CH_3$ | $-CH_3$ | $-(CH_2)_2CN$ | $-O-CH_2-$ | 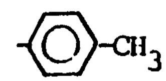 | 199–200 | 59 % (C) |
| 36 | $-CH_3$ | $-CH_3$ | $-CH_3$ | $-(CH_2)_2CN$ | $-O-CH_2$ | 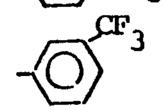 | 152–154 | 61 % (C) |
| 37 | $-CH_3$ | $-CH_3$ | $-CH_3$ | $-(CH_2)_2CN$ | $-S-CH_2-$ | 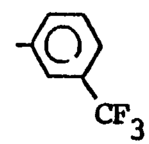 | amorph | 90 % (C) |
| 38 | 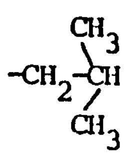 | $-CH_3$ | $-CH_3$ | $-(CH_2)_2CN$ | $-O-CH_2-$ | 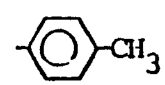 | 177–178 | 60 % (C) |

| Bei-spiel | $R^3$ | $R^4$ | $R^1$ | $R^2$ | X-B | Y | FP/°C | Ausbeute (Darstellungs-verfahren) |
|---|---|---|---|---|---|---|---|---|
| 39 | 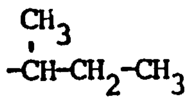 | $-CH_3$ | $-CH_3$ | $-(CH_2)_2CN$ | $-O-CH_2-$ | 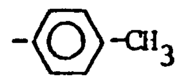 | 183-185 | 50 % (C) |
| 40 | 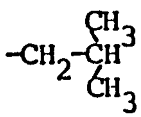 | $-CH_3$ | $-CH_3$ | $-(CH_2)_2CN$ | $-O-CH_2-$ | 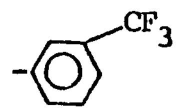 | 175-178 | 67 % (C) |
| 41 | 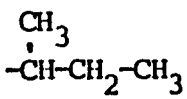 | $-CH_3$ | $-CH_3$ | $-(CH_2)_2CN$ | $-O-CH_2-$ | 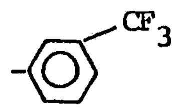 | 130-131 | 41 % (C) |
| 42 | $-CH_3$ | $-CH_3$ | $-CH_3$ | $-(CH_2)_2CN$ | $-O-CH_2-$ | 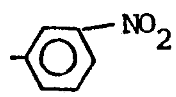 | 178-183 | 70 % (C) |
| 43 | $-C_2H_5$ | $-CH_3$ | $-CH_3$ | $-(CH_2)_2CN$ | $-O-CH_2$ | 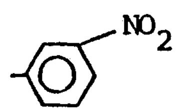 | 172-174 | 62 % (C) |
| 44 | $-CH_3$ | $-CH_3$ | $-CH_3$ | $-(CH_2)_2CN$ | $-O-CH_2-$ | 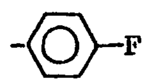 | 193-196 | 53 % (C) |
| 45 | $-C_2H_5$ | $-CH_3$ | $-CH_3$ | $-(CH_2)_2CN$ | $-O-CH_2-$ | 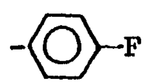 | 198-200 | 48 % (C) |

| Beispiel | $R^3$ | $R^4$ | $R^1$ | $R^2$ | X-B | Y | FP/°C | Ausbeute (Darstellungsverfahren) |
|---|---|---|---|---|---|---|---|---|
| 46 | $-CH_3$ | $-CH_3$ | $-CH_3$ | $-(CH_2)_2CN$ | $-S-CH_2-CH_2-CH_2$ | 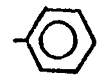 | amorph | 95 % (C) |
| 47 | $-CH_3$ | $-CH_3$ | $-CH_3$ | $-(CH_2)_2CN$ | $-O-CH_2-$ | 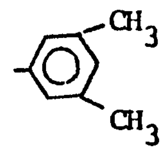 | 168-170 | 50 % (C) |
| 48 | $-(CH_2)_3-CH_3$ | $-CH_3$ | $-CH_3$ | $-(CH_2)_2CN$ | $-O-CH_2$ | 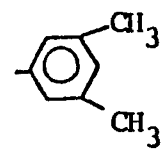 | 160-161 | 42 % (C) |
| 49 | $-CH_3$ | $-CH_3$ | $-CH_3$ | $-(CH_2)_2CN$ | $-O-CH_2$ | 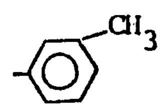 | 150-152 | 49 % (C) |
| 50 | $-(CH_2)_3CH_3$ | $-CH_3$ | $-CH_3$ | $-(CH_2)_2CN$ | $-O-CH_2$ | 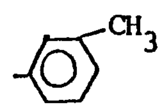 | 173-176 | 44 % (C) |
| 51 | $-CH_3$ | $-CH_3$ | $-CH_3$ | $-(CH_2)_2CN$ | $-O-CH_2$ | 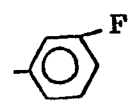 | 147-149 | 43 % (C) |

| Bei-spiel | $R^3$ | $R^4$ | $R^1$ | $R^2$ | X-B | Y | FP/°C | Ausbeute (Darstellungs-verfahren) |
|---|---|---|---|---|---|---|---|---|
| 52 | $-(CH_2)_3CH_3$ | $-CH_3$ | $-CH_3$ | $-(CH_2)_2CN$ | $-O-CH_2-$ | 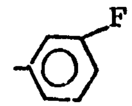 | 182-185 | 53 % (C) |
| 53 | $-CH_3$ | $-CH_3$ | $-CH_3$ | $-(CH_2)_2CN$ | $-O-CH_2-$ | 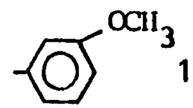 | 138-142 | 56 % (C) |
| 54 | $-(CH_2)_3-CH_3$ | $-CH_3$ | $-CH_3$ | $-(CH_2)_2CN$ | $-O-CH_2-$ | 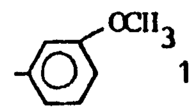 | 128-131 | 43 % (C) |
| 55 | $-CH_3$ | $-CH_3$ | $-CH_3$ | $-(CH_2)_2CN$ | $-O-CH_2-$ | 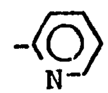 | amorph* RF-Wert = 0,14 | 88 % (C) |
| 56 | $-(CH_2)_3CH_3$ | $-CH_3$ | $-CH_3$ | $-(CH_2)_2CN$ | $-O-CH_2-$ | 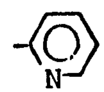 | amorph* RF-Wert = 0,16 | 95 % (C) |
| 57 | $-CH_3$ | $-CH_3$ | $-CH_3$ | $-(CH_2)_2CN$ | $-OCH_2-$ | -S- 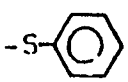 | amorph* RF-Wert = 0,62 | 93 % (C) |

*) DC Fertigplatten Kieselgel 60 $F_{254}$ Merck/Fließmittel $CHCl_3$-Essigester = 3:1

| Beispiel | $R^3$ | $R^4$ | $R^1$ | $R^2$ | X-B | Y | FP/°C | Ausbeute (Darstellungsverfahren) |
|---|---|---|---|---|---|---|---|---|
| 58 | $-CH_3$ | $-CH_3$ | $-CH_3$ | $-CH_3$ | $-S-CH_2-$ | 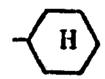 | 142-145 | 30 % (D) |
| 59 | $-C_2H_5$ | $-CH_3$ | $-CH_3$ | $-C_2H_5$ | $-S-CH_2-$ | 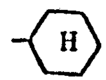 | 209-211 | 55 % (D) |
| 60 | $-(CH_2)_2OCH_3$ | $-CH_3$ | $-CH_3$ | $-(CH_2)_2OCH_3$ | $-S-CH_2-$ | 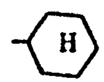 | 84-87 | 25 % (D) |
| 61 | $-CH_3$ | $-CH_3$ | $-CH_3$ | $-CH_3$ | -O- | $-SO_2-$ 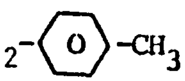 | amorph | 40 % (D) |
| 62 | $-C_2H_5$ | $-CH_3$ | $-CH_3$ | $-C_2H_5$ | -O- | $-SO_2-$ 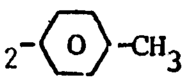 | 145 | 38 % (D) |
| 63 | $-CH_3$ | $-CH_3$ | $-CH_3$ | $-CH_3$ | $-OCH_2-$ | 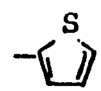 | 162 | 18 % (D) |
| 64 | $-CH_3$ | $-CH_3$ | $-CH_3$ | $-CH_3$ | $-OCH_2-$ | 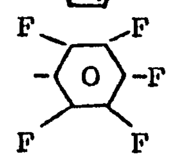 | 160 | 20 % (D) |
| 65 | $-CH_3$ | $-CH_3$ | $-CH_3$ | $-CH_3$ | $-OCH_2-$ | 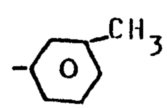 | 160 | 20 % (D) |

| Beispiel | $R^3$ | $R^4$ | $R^1$ | $R^2$ | X - B | Y | FP/°C | Ausbeute (Darstellungsverfahren) |
|---|---|---|---|---|---|---|---|---|
| 66 | $-CH_3$ | $-CH_3$ | $-CH_3$ | $-CH_3$ | $-OCH_2-$ | 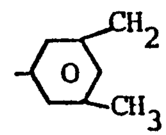 | 188 | 35 % (D) |
| 67 | $-CH_3$ | $-CH_3$ | $-CH_3$ | $-CH_3$ | $-OCH_2$ | 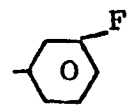 | 132 | 42 % (D) |
| 68 | $-CH_3$ | $-CH_3$ | $-CH_3$ | $-CH_3$ | $-OCH_2$ | 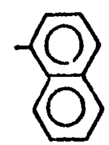 | 153 | 20 % (D) |
| 69 | $-CH_3$ | $-CH_3$ | $-CH_3$ | $-CH_3$ | -O- | $-SO_2-$ 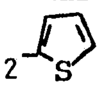 | 132 | 20 % (D) |

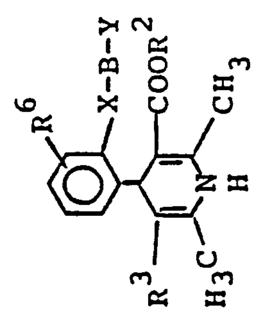

| Beispiel | $R^3$ | $R^2$ | X - B | Y | $R^6$ | FP/°C | Ausbeute (Darstellungsverfahren) |
|---|---|---|---|---|---|---|---|
| 70 | $-CH_3$ | $-CH_3$ | $-OCH_2-$ | 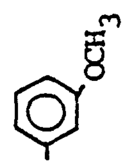 | $3-OCH_3$ | 189 | 31 % (D) |
| 71 | $-C_2H_5$ | $-C_2H_5$ | $-S-CH_2-$ | 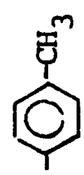 | $5-NO_2$ | 175–178 | 45 % (D) |

**Patentansprüche**

1. 1,4-Dihydropyridine der allgemeinen Formel I

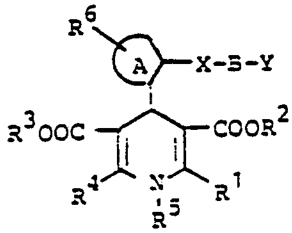

(I

in welcher

$R^1$ und $R^4$ gleich oder verschieden sind und für Wasserstoff oder einen geradkettigen oder verzweigten Alkylrest mit bis zu 6 Kohlenstoffatomen stehen, der gegebenenfalls 1 oder 2 gleiche oder verschiedene Heterokettenglieder aus der Gruppe O, CO, NH oder N-Alkyl mit 1 bis 4 Kohlenstoffatomen enthält und der gegebenenfalls substituiert ist durch Halogen, Nitro, Cyano, Hydroxy, Amino oder Phenyl,

$R^2$ und $R^3$ gleich oder verschieden sind und jeweils einen verzweigten oder unverzweigten Alkyloder Alkenylrest mit bis zu 6 Kohlenstoffatomen stehen, der gegebenenfalls substituiert ist durch Halogen, Hydroxy, Cyano, Amido, Monoalkylamino, Dialkylamino oder Alkyl-benzylamino, wobei die genannten Alkylreste jeweils 1 bis 4 Kohlenstoffatome enthalten,

$R^5$ für Wasserstoff, Benzyl oder einen geradkettigen oder verzweigten Alkylrest mit bis zu 6 Kohlenstoffatomen steht, der gegebenenfalls substituiert ist durch Fluor, Chlor oder Alkoxy mit 1 bis 4 Kohlenstoffatomen,

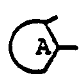

für Phenyl, Naphthyl, Thirnyl, Furyl oder Pyridyl steht,

$R^6$ für einen oder 2 gleiche oder verschiedene Substituenten aus der Gruppe Wasserstoff, Alkyl, Alkoxy mit jeweils 1 bis 4 Kohlenstoffatomen, Fluor, Chlor, Brom, Cyano, Trifluormethyl, Carboxy, Amino, Hydroxy, Alkylamino oder Halogenalkoxy mit jeweils 1 bis 4 Kohlenstoffatomen steht,

X für ein Brückenglied aus der Gruppe O, S oder SO steht,

B für eine geradkettige oder verzweigte Alkylengruppe mit 1 bis 4 Kohlenstoffatomen steht und

Y für Phenyl, Pyridyl, Cycloalkyl mit 3 bis 7 Kohlenstoffatomen oder Alkenyl mit 6 bis 10 Kohlenstoffatomen steht, wobei diese Reste

entweder direkt mit B verbunden sind oder über ein Heteroatom aus der Gruppe O, S, SO, $SO_2$, NH oder N-Alkyl mit 1 bis 4 Kohlenstoffatomen, mit B verbunden sind und wobei diese Reste gegebenenfalls 1 oder 2 gleiche oder verschiedene Substituenten aus der Gruppe Nitro, Halogen, Trifluormethyl, Cyano, Hydroxy, Phenyl, Alkyl und Alkoxy mit jeweils 1 bis 4 Kohlenstoffatomen enthalten, sowie ihre pharmazeutischen unbedenklichen Additionssalze.

2. Verfahren zur Herstellung von Verbindungen der allgemeinen Formel I

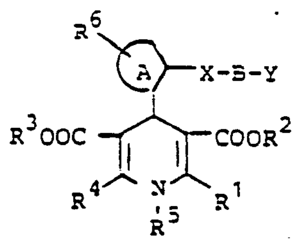

(I)

in welcher

$R^1$ und $R^4$ gleich oder verschieden sind und für Wasserstoff oder einen geradkettigen oder verzweigten Alkylrest mit bis zu 6 Kohlenstoffatomen stehen, der gegebenenfalls 1 oder 2 gleiche oder verschiedene Heterokettenglieder aus der Gruppe O, CO, NH oder N-Alkyl mit 1 bis 4 Kohlenstoffatomen enthält und der gegebenenfalls substituiert ist durch Halogen, Nitro, Cyano, Hydroxy, Amino oder Phenyl,

$R^2$ und $R^3$ gleich oder verschieden sind und jeweils einen verzweigten oder unverzweigten Alkyloder Alkenylrest mit bis zu 6 Kohlenstoffatomen stehen, der gegebenenfalls substituiert ist durch Halogen, Hydroxy, Cyano, Amido, Monoalkylamino, Dialkylamino oder Alkyl-benzylamino, wobei die genannten Alkylreste jeweils 1 bis 4 Kohlenstoffatome enthalten,

$R^5$ für Wasserstoff, Benzyl oder einen geradkettigen oder verzweigten Alkylrest mit bis zu 6 Kohlenstoffatomen steht, der gegebenenfalls substituiert ist durch Fluor, Chlor oder Alkoxy mit 1 bis 4 Kohlenstoffatomen,

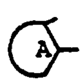

für Phenyl, Naphthyl, Thienyl, Furyl oder Pyridyl steht,

$R^6$ für einen oder 2 gleiche oder verschiedene Substituenten aus der Gruppe Wasserstoff, Alkyl, Alkoxy mit jeweils 1 bis 4 Kohlenstoffatomen, Fluor, Chlor, Brom, Cyano, Trifluormethyl, Carboxy, Amino, Hydroxy, Alkylamino oder Halogenalkoxy mit jeweils 1 bis 4 Kohlenstoffatomen steht,

X für ein Brückenglied aus der Gruppe O, S oder SO steht,

B für eine geradkettige oder verzweigte Alkylengruppe mit 1 bis 4 Kohlenstoffatomen steht und

Y für Phenyl, Pyridyl, Cycloalkyl mit 3 bis 7 Kohlenstoffatomen oder Alkenyl mit 6 bis 10 Kohlenstoffatomen steht, wobei diese Reste entweder direkt mit B verbunden sind oder über ein Heteroatom aus der Gruppe O, S, SO, $SO_2$, NH oder N-Alkyl mit 1 bis 4 Kohlenstoffatomen, mit B verbunden sind und wobei diese Reste gegebenenfalls 1 oder 2 gleiche oder verschiedene Substituenten aus der Gruppe Nitro, Halogen, Trifluormethyl, Cyano, Hydroxy, Phenyl, Alkyl und Alkoxy mit jeweils 1 bis 4 Kohlenstoffatomen enthalten,

dadurch gekennzeichnet, daß man

A) Yliden-β-ketoester der allgemeinen Formel (II)

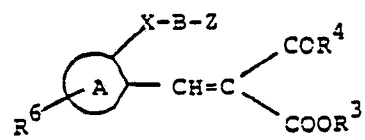

(II)

in welcher

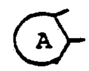

,

$R^3$, $R^4$, $R^6$, X, B und Z die oben angegebene Bedeutung haben,

mit Enaminocarbonsäureestern der allgemeinen Formel (III)

$$R^1-C=CH-COOR^2 \quad (III)$$
$$R^5NH$$

in welcher

$R^1$, $R^2$ und $R^5$ die oben angegebene Bedeutung haben,

gegebenenfalls in Gegenwart inerter organischer Lösungsmittel bei Temperaturen zwischen 20 und 150° C umsetzt, oder

B) Yliden-β-ketoester der allgemeinen Formel (II)

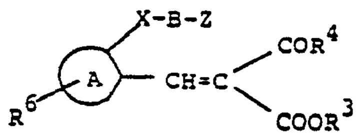

(II)

in welcher

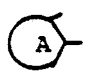

,

$R^3$, $R^4$, $R^6$, X, B und Z die oben angegebene Bedeutung haben,

mit Aminen der allgemeinen Formel (IV)

$R^5$-$NH_2$ (IV)

in welcher

$R^5$ die oben angegebene Bedeutung besitzt und β-Ketocarbonsäureestern der allgemeinen Formel (V)

$R^1$-CO-$CH_2$-$COOR^2$ (V)

in welcher

$R^1$ und $R^2$ die oben angegebene Bedeutung haben,

gegebenenfalls in Gegenwart inerter organischer Lösungsmittel bei Temperaturen zwischen 20 und 150° C umsetzt, oder

C) Aldehyde der allgemeinen Formel (VI)

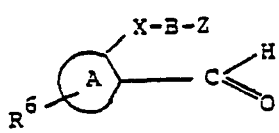

(VI)

in welcher

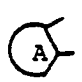

,

$R^6$, X, B und Z die oben angegebene Bedeutung haben,

mit Enaminocarbonsäureestern der allgemeinen Formel (III)

$R^1$-C=CH-$COOR^2$ (III)

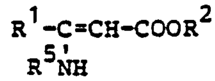

(III)

$R^5NH$

in welcher

$R^1$, $R^2$ und $R^5$ die oben angegebene Bedeutung haben,

und β-Ketocarbonsäureestern der allgemeinen Formel (VII)

$R^4$-CO-$CH_2$-$COOR^3$ (VII)

in welcher

$R^3$ und $R^4$ die oben angegebene Bedeutung haben,

gegebenenfalls in Gegenwart inerter organischer Lösungsmittel bei Temperaturen zwischen 20 und 150° C umsetzt, oder

D) Aldehyde der allgemeinen Formel (VI)

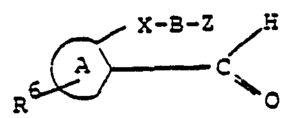

(VI)

in welcher

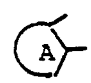

,

$R^6$, X, B und Z die oben angegebene Bedeutung haben,

mit Aminen der allgemeinen Formel (IV)

$R^5$-$NH_2$ (IV)

in welcher

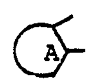

,

$R^6$, X, B und Z die oben angegebene Bedeutung haben,

mit Aminen der allgemeinen Formel (IV)

$R^5$-$NH_2$ (IV)

in welcher

$R^5$ die oben angegebene Bedeutung hat,

und zwei äquivalenten β-Ketoester der allgemeinen Formel (VII)

$R^4$-CO-$CH_2$-$COOR^3$ (VII)

in welcher

$R^3$ und $R^4$ die oben angegebene Bedeutung haben,

gegebenenfalls in Gegenwart inerter organischer Lösungsmittel bei Temperaturen zwischen 20 und 150° C umsetzt.

3. Verbindungen der allgemeinen Formel I gemäß Anspruch 1 zur Verwendung bei der Bekämpfung von Erkrankungen.

4. Verbindungen der allgemeinen Formel I gemäß Anspruch 1 zur Verwendung bei der Bekämpfung von Kreislauferkrankungen.

5. Arzneimittel enthaltend mindestens eine

Verbindung der allgemeinen Formel I gemäß Anspruch 1.

6. Verfahren zur Herstellung von Arzneimitteln, dadurch gekennzeichnet, daß man Verbindungen der allgemeinen Formel I gemäß Anspruch 1 gegebenenfalls unter Verwendung von üblichen Hilfs- und Trägerstoffen in eine geeignete Applikationsform überführt.

7. Verwendung von Verbindungen der allgemeinen Formel I gemäß Anspruch 1 bei der Herstellung von Kreislaufmitteln.

8. Verwendung von Verbindungen der allgemeinen Formel I gemäß Anspruch 1 bei der Herstellung von blutdrucksenkenden Mitteln.

**Claims**

1. 1,4-Dihydropyridines of the general formula I

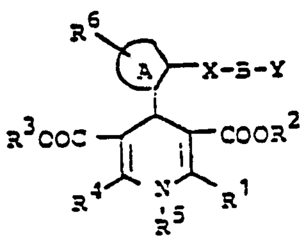

(I)

in which

$R^1$ and $R^4$ are identical or different and represent hydrogen or a straight-chain or branched alkyl radical with up to 6 carbon atoms which optionally contains 1 or 2 identical or different hetero chain members from the group comprising O, CO, NH or N-alkyl with 1 to 4 carbon atoms and which is optionally substituted by halogen, nitro, cyano, hydroxyl, amino or phenyl,

$R^2$ and $R^3$ are identical or different and each represent a branched or unbranched alkyl or alkenyl radical with up to 6 carbon atoms which is optionally substituted by halogen, hydroxyl, cyano, amido, monoalkylamino, dialkylamino or alkyl-benzylamino, the alkyl radicals mentionad each containing 1 to 4 carbon atoms,

$R^5$ represents hydrogen, benzyl or a straight-chain or branchéd alkyl radical with up to 6 carbon atoms which is optionally substituted by fluorine, chlorine or alkoxy with 1 to 4 carbon atoms,

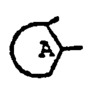

epresents phenyl, naphthyl, thienyl, furyl or pyridyl,

$R^6$ represents one or 2 identical or different substituents from the group comprising hydrogen, alkyl and alkoxy with in each case 1 to 4 carbon atoms, fluorine, chlorine, bromine, cyano, trifluoromethyl, carboxyl, amino, hydroxyl, alkylamino or halogenoalkoxy with in each case 1 to 4 carbon atoms,

X represents a bridging member from the group comprising O, S or SO,

B represents a straight-chain or branched alkylene group with 1 to 4 carbon atoms and

Y represents phenyl, pyridyl, cycloalkyl with 3 to 7 carbon atoms or alkenyl with 6 to 10 carbon atoms, these radicals being either directly bonded to B or being bonded to B via a hetero atom from the group comprising O, S, SO, $SO_2$, NH or N-alkyl with 1 to 4 carbon atoms and these radicals optionally containing 1 or 2 identical or different substituents from the group comprising nitro, halogen, trifluoromethyl, cyano, hydroxyl, phenyl, alkyl and alkoxy with in each case 1 to 4 carbon atoms,

and their pharmaceutically acceptable addition salts.

2. Process for the preparation of compounds of the general formula I

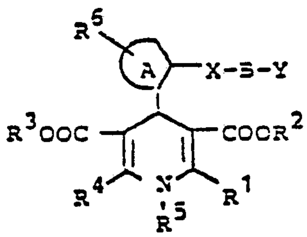

(I)

in which

$R^1$ and $R^4$ are identical or different and represent hydrogen or a straight-chain or branched alkyl radical with up to 6 carbon atoms which optionally contains 1 or 2 identical or different hetero chain members from the group comprising O, CO, NH or N-alkyl with 1 to 4 carbon atoms and which is optionally substituted by halogen, nitro, cyano, hydroxyl, amino or phenyl,

$R^2$ and $R^3$ are identical or differant and each represent a branched or unbranched alkyl or alkenyl radical with up to 6 carbon atoms which is optionally substituted by halogen, hydroxyl, cyano, amido, monoalkylamino, dialkylamino or alkyl-benzylamino, the alkyl radicals mentioned each containing 1 to 4 carbon atoms,

$R^5$ represents hydrogen, benzyl or a straight-chain or branched alkyl radical with up to 6 carbon atoms which is optionally substituted by fluorine, chlorine or alkoxy with 1 to 4 carbon atoms,

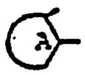

epresents phenyl, naphthyl, thienyl, furyl or pyridyl,

$R^6$ represents one or 2 identical or different substituents from the group comprising hydrogen, alkyl and alkoxy with in each case 1 to 4 carbon atoms, fluorine, chlorine, bromine, cyano, trifluoromethyl, carboxyl, amino, hydroxyl, alkylamino or halogenoalkoxy with in each case 1 to 4 carbon atoms,

X represents a bridging member from the group comprising O, S or SO,

B represents a straight-chain or branched alkylene group with 1 to 4 carbon atoms and

Y represents phenyl, pyridyl, cyclcalkyl with 3 to 7 carbon atoms or alkenyl with 6 to 10 carbon atoms, these radicals being either directly bonded to B or being bonded to B via a hetero atom from the group comprising O, S, SO, $SO_2$, NH or N-alkyl with 1 to 4 carbon atoms and these radicals optionally containing 1 or 2 identical or different substituents from the group comprising nitro, halogen, trifluoromethyl, cyano, hydroxyl, phenyl, alkyl and alkoxy with in each case 1 to 4 carbon atoms,

characterised in that

A) ylidene-β-ketoesters of the general formula (II)

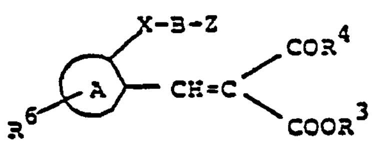
(II)

in which

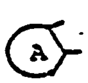 ,

$R^3$, $R^4$, $R^6$, X, B and Z have the abovementioned meaning,

are reacted with enaminocarboxylic acid esters of the general formula (III)

$R^1$-C=CH-COOR$^2$ (III)

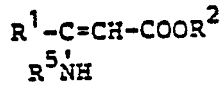
(III)

$R^5$NH

in which

$R^1$, $R^2$ and $R^5$ have the abovementioned meaning,

if appropriate in the presence of inert organic solvents at temperatures between 20 and 150°C, or

B) ylidene-β-ketoesters of the general formula (II)

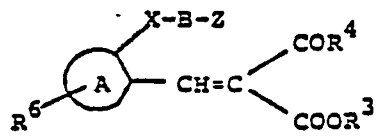
(II)

in which

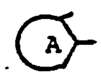 ,

$R^3$, $R^4$, $R^6$, X, B and Z have the abovementioned meaning, are reacted with amines of the general formula (IV)

$R^5$-NH$^2$ (IV)

in which

$R^5$ has the abovementioned meaning and β-ketocarboxylic acid esters of the general formula (V)

$R^1$-CO-$CH_2$-COOR$^2$ (V)

in which

$R^1$ and $R^2$ have the abovementioned meaning, if appropriate im the presence of inert organic solvents at temperatures between 20 and 150°C, or

c) aldehydes of the general formula (VI)

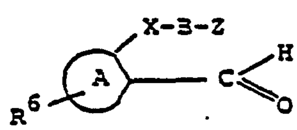
(VI)

in which

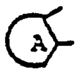 ,

$R^6$, X, B and Z have the abovementioned meaning, are reacted with enaminocarboxylic acid esters of the general formula (III)

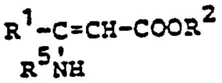 (III)

in which

$R^1$, $R^2$ and $R^5$ have the abovementioned meaning, and β-ketocarboxylic acid esters of the general formula (VII)

$R^4$-CO-$CH_2$-$COOR^3$ (VII)

in which

$R^3$ and $R^4$ have the abovementioned meaning, if appropriate in the presence of inert organic solvents at temperatures between 20 and 150° C, or

D) aldehydes of the general formula (VI)

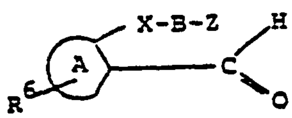 (VI)

in which

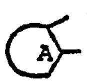 ,

$R^6$, X, B and Z have the abovementioned meaning, are reacted with amines of the general formula (IV)

$R^5$-$NH_2$ (IV)

in which

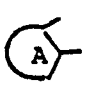 ,

$R^6$, X, B and Z have the abovementioned meaning, with amines of the general formula (IV)

$R^5$-$NH_2$ (IV)

in which

$R^5$ has the abovementioned meaning, and two equivalents of β-ketoesters of the general formula (VII)

$R^4$- CO-$CH_2$-$COOR^3$ (VII)

in which

$R^3$ and $R^4$ have the abovementioned meaning, if appropriate in the presence of inert organic solvents at temperatures between 20 and 150° C.

3. Compounds of the general formula I according to Claim 1 for use in combating diseases.

4. Compounds of the general formula I according to Claim 1 for use in combating circulatory diseases.

5. Medicaments containing at least one compound of the general formula I according to Claim 1.

6. Process for the preparation of medicaments, characterised in that compounds of the general formula I according to Claim 1 are converted into a suitable form of administration, if appropriate using customary auxiliaries and excipients.

7. Use of compounds of the general formula I according to Claim 1 in the preparation of circulatory agents.

8. Use of compounds of the general formula I according to Claim 1 in the preparation of hypotensive agents.

**Revendications**

1. 1,4-Dihydropyridines de formule générale I

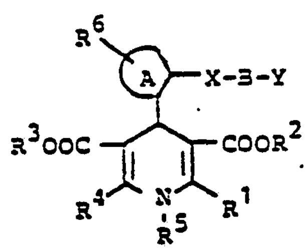 (I)

dans laquelle

$R^1$ et $R^4$, ayant des significations identiques ou différentes, représentent chacun un atome d'hydrogène ou un groupe alkyle à chaîne droite ou ramifiée contenant jusqu'à 6 atomes de carbone, qui peut contenir, le cas échéant, 1 ou 2 chaînons hétérogènes identiques ou différents du groupe formé par O, CO, NH ou N-alkyle contenant 1 à 4 atomes de carbone et est, le cas échéant, substitué par des halogènes, des groupes nitro, cyano, hydroxy, amino ou phényle,

$R^2$ et $R^3$, ayant des significations identiques ou différentes, représentent chacun un groupe alkyle ou alcényle droit ou ramifié contenant jusqu'à 6 atomes de carbone, éventuellement substitué par des halogènes, des groupes hydroxy, cyano, amido, monoalkylamino, dialkylamino ou alkyl-benzylamino, les groupes

alkyle mentionnés contenant chacun 1 à 4 atomes de carbone,

$R^5$ représente l'hydrogène, un groupe benzyle ou un groupe alkyle à chaîne droite ou ramifiée contenant jusqu'à 6 atomes de carbone, éventuellement substitué par le fluor, le chlore ou des groupes alcoxy en $C_1$-$C_4$,

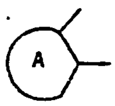

eprésente un groupe phényle, naphtyle, thiényle, furyle ou pyridyle,

$R^6$ représente un ou deux substituants identiques ou différents du groupe formé par l'hydrogène, les groupes alkyle, alcoxy contenant chacun 1 à 4 atomes de carbone, le fluor, le chlore, le brome, les groupes cyano, trifluorométhyle, carboxy, amino, hydroxy, alkylamino ou halogénoalcoxy contenant chacun 1 à 4 atomes de carbone,

X représente un pont du groupe formé par O, S ou SO,

B représente un groupe alkylène à chaîne droite ou ramifiée en $C_1$-$C_4$, et

Y représente un groupe phényle, pyridyle, cycloalkyle en $C_3$-$C_7$ ou alcényle en $C_6$-$C_{10}$, ces groupes pouvant être soit reliés directement avec B, soit reliés à B par l'intermédiaire d'un hétéroatome du groupe formé par O, S, SO, $SO_2$, NH ou N-alkyle en $C_1$-$C_4$, ces groupes pouvant porter, le cas échéant, un ou deux substituants identiques ou différents de la classe formée par les groupes nitro, les halogènes, les groupes trifluorométhyle, cyano, hydroxy, phényle, alkyle et alcoxy contenant chacun 1 à 4 atomes de carbone,

et leurs sels d'addition acceptables pour l'usage pharmaceutique.

2. Procédé de préparation des composés de formule générale I

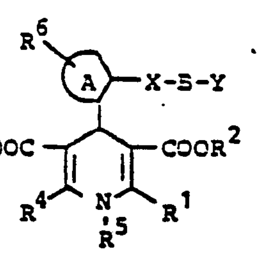

(I)

dans laquelle

$R^1$ et $R^4$, ayant des significations identiques ou différentes, représentent chacun l'hydrogène ou un groupe alkyle à chaîne droite ou ramifiée contenant jusqu'à 6 atomes de carbone, qui peut contenir, le cas échéant, 1 ou 2 chaînons hétérogènes identiques ou différents du groupe formé par O, CO, NH ou N-alkyle en $C_1$-$C_4$ et qui est, le cas échéant, substitué par des halogènes, des groupes nitro, cyano, hydroxy, amino ou phényle,

$R^2$ et $R^3$, ayant des significations identiques ou différentes, représentent chacun un groupe alkyle ou alcényle droit ou ramifié contenant jusqu'à 6 atomes de carbone, éventuellement substitué par des halogènes, des groupes hydroxy, cyano, amido, monoalkylamino, dialylamino ou alkyl-benzylamino, les groupes alkyle mentionnés contenant chacun 1 à 4 atomes de carbone,

$R^5$ représente l'hydrogène un groupe benzyle ou un groupe alkyle à chaîne droite ou ramifiée contenant jusqu'à 6 atomes de carbone, éventuellement substitué par le fluor, le chlore ou des groupes alcoxy en $C_1$-$C_4$,

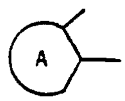

eprésente un groupe phényle, naphtyle, thiényle, furyle ou pyridyle,

$R^6$ représente un ou deux substituants identiques ou différents du groupe formé par l'hydrogène, les groupes alkyle, alcoxy contenant chacun 1 à 4 atomes de carbone, le fluor, le chlore, le brome, les groupes cyano, trifluorométhyle, carboxy, amino, hydroxy, alkylamino ou halogénoalcoxy contenant chacun 1 à 4 atomes de carbone,

X représente un pont du groupe O, S ou SO,

B représente un groupe alkylène à chaîne droite ou ramifiée en $C_1$-$C_4$, et

Y représente un groupe phényle, pyridyle, cycloalkyle en $C_3$-$C_7$ ou alcényle en $C_6$-$C_{10}$, ces groupes pouvant être soit reliés directement à B, soit reliés à B par l'intermédiaire d'un hétéroatome du groupe O, S, SO, $SO_2$, NH ou N-alkyle en $C_1$-$C_4$, et ces groupes pouvant, le cas échéant, porter un ou deux substituants identiques ou différents de la classe des groupes nitro, des halogènes, des groupes trifluorométhyle, cyano, hydroxy, phényle, alkyle et alcoxy contenant chacun 1 à 4 atomes de carbone,

caractérisé en ce que:

A) on fait réagir des ylidène-β-cétoesters de formule générale II

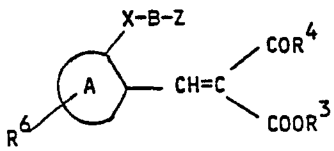

(II)

dans laquelle

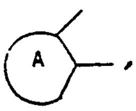

$R^3$, $R^4$, $R^6$, X, B et Z ont les significations indiquées ci-dessus,

avec des esters d'acides énaminocarboxyliques de formule générale III

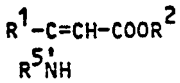

(III)

dans laquelle

$R^1$, $R^2$ et $R^5$ ont les significations indiquées ci-dessus, éventuellement en présence de solvants organiques inertes à des températures de 20 à 150° C, ou bien

B) on fait réagir des ylidène-β-cétoesters de formule générale II

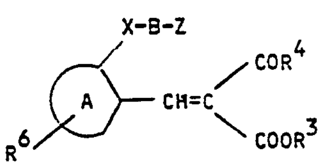

(II)

dans laquelle

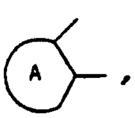

$R^3$, $R^4$, $R^6$, X, B et Z ont les significations indiquées ci-dessus,

avec des amines de formule générale IV

$R^5$-$NH_2$ (IV)

dans laquelle

$R^5$ a les significations indiquées ci-dessus,

et des esters d'acides β-cétocarboxyliques de formule générale V

$R^1$-CO-$CH_2$-$COOR^2$ (V)

dans laquelle

$R^1$ et $R^2$ ont les significations indiquées ci-dessus, éventuellement en présence de solvants organiques inertes à des températures de 20 à 150° C, ou bien

C) on fait réagir des aldéhydes de formule générale VI

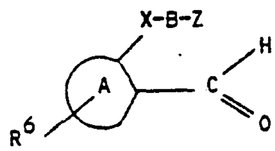

(VI)

dans laquelle

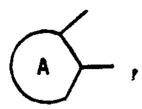

$R^6$, X, B et Z ont les significations indiquées ci-dessus, avec des esters d'acides énaminocarboxyliques de formule générale III

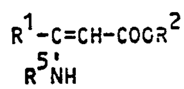

(III)

dans laquelle

$R^1$, $R^2$ et $R^5$ ont les significations indiquées ci-dessus, et des esters d'acides β-cétocarboxyliques de formule générale VII

$R^4$-CO-$CH_2$-$COOR^3$ (VII)

dans laquelle

$R^3$ et $R^4$ ont les significations indiquées ci-dessus,

éventuellement en présence de solvants organiques, à des températures de 20 à 150° C, ou bien

D) on fait réagir des aldéhydes de formule générale VI

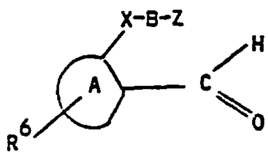 (VI)

dans laquelle

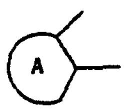 ,

$R^6$, X, B et Z ont les significations indiquées ci-dessus, avec des amines de formule générale IV

$R^5$-$NH_2$ (IV)

dans laquelle

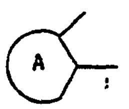 ,

$R^6$, X, B et Z ont les significations indiquées ci-dessus, avec des amines de formule générale IV

$R^5$-$NH_2$ (IV)

dans laquelle

$R^5$ a les significations indiquées ci-dessus,

et deux équivalents de β-cétoesters de formule générale VII

$R^4$-CO-$CH_2$-$COOR^3$ (VII)

dans laquelle

$R^3$ et $R^4$ ont les significations indiquées ci-dessus,

éventuellement en présence de solvants organiques inertes à des températures de 20 à 150°C.

3. Composés de formule générale I selon la revendication 1 pour l'utilisation dans le traitement de maladies.

4. Composés de formule générale I selon la revendication 1 pour l'utilisation dans le traitement de maladies de la circulation.

5. Médicament contenant au moins un composé de formule générale I selon la revendication 1.

6. Procédé de préparation de médicaments, caractérisé en ce que l'on met des composés de formule générale I selon la revendication 1, éventuellement en utilisant des produits auxiliaires et véhicules usuels, sous une forme d'administration appropriée.

7. Utilisation des composés de formule générale I selon la revendication 1 dans la préparation d'agents circulatoires.

8. Utilisation des composés de formule générale I selon la revendication 1 dans la préparation d'agents abaissant la pression sanguine.